(19) Europäisches Patentamt

European Patent Office

Office européen des brevets

(11) **EP 1 632 221 A1**

(12) **DEMANDE DE BREVET EUROPEEN**

(43) Date de publication:
08.03.2006 Bulletin 2006/10

(51) Int Cl.:
*A61K 8/87* (2006.01)        *A61Q 5/00* (2006.01)
*A61Q 19/00* (2006.01)

(21) Numéro de dépôt: 05291362.1

(22) Date de dépôt: 24.06.2005

(84) Etats contractants désignés:
AT BE BG CH CY CZ DE DK EE ES FI FR GB GR
HU IE IS IT LI LT LU MC NL PL PT RO SE SI SK TR
Etats d'extension désignés:
AL BA HR LV MK YU

(30) Priorité: 01.07.2004 FR 0407304

(71) Demandeur: L'OREAL
75008 Paris (FR)

(72) Inventeurs:
• **Samain, Henri**
91570 Bievres (FR)
• **Rollat-Corvol, Isabelle**
75017 Paris (FR)
• **Gawtrey, Jonathan**
92100 Boulogne (FR)

(74) Mandataire: **Bourdeau, Françoise**
L'OREAL - D.I.P.I.
25-29 Quai Aulagnier
92600 Asnières (FR)

(54) **Composition cosmétique non rincée comprenant des polymères filmogènes élastomériques, son utilisation pour le conditionnement des matières kératiniques**

(57) L'invention a pour objet une composition cosmétique non rincée comprenant, dans un milieu cosmétiquement acceptable, au moins un polymère filmogène élastomérique choisi de telle sorte que le film obtenu par séchage de ce polymère, en milieu aqueux ou alcoolique, à température ambiante et à un taux d'humidité relative de 55 %, présente un profil mécanique défini par au moins :

(a) un taux d'allongement à la rupture ($\varepsilon_r$) supérieur ou égal à 800 %,

(b) une recouvrance instantanée ($R_i$) au moins égale à 75 %, après un allongement de 150 %,
(c) une recouvrance ($R_{300}$) à 300 secondes supérieure à 80 %.

Cette composition permet notamment le conditionnement des fibres kératiniques telles que les cheveux.

**Description**

**DOMAINE DE L'INVENTION**

L'invention a pour objet une composition cosmétique comprenant, dans un milieu cosmétiquement acceptable, un polymère filmogène aux caractéristiques élastomériques particulières. Elle vise également l'utilisation de cette composition pour la formulation de produits non rincés, destinés aux matières kératiniques humaines comme la peau, les ongles et les fibres kératiniques, ces produits étant notamment des produits de soin des cheveux, de la peau, des cils tels que des produits de protection et de réparation des cheveux, des produits de coiffage.

L'invention a encore pour objet un procédé de coiffage, de mise en forme ou de maintien des fibres kératiniques et plus spécialement des cheveux à l'aide de cette composition.

La fixation de la coiffure est un élément important du coiffage, et implique le maintien d'une forme qui a déjà été réalisée. Selon l'invention, le terme "composition de coiffage" concerne tout type de composition capillaire pouvant être utilisé pour effectuer le coiffage.

**ARRIERE PLAN TECHNOLOGIQUE**

Parmi les produits capillaires pour la fixation des cheveux les plus répandus sur le marché de la cosmétique, on peut citer les compositions à pulvériser en aérosol ou en flacon pompe tels que les laques, les sprays ou les mousses, essentiellement constituées d'une solution le plus souvent alcoolique ou hydroalcoolique et d'un polymère filmogène soluble dans l'eau ou dans l'alcool, en mélange avec divers adjuvants cosmétiques ou encore les produits à appliquer à la main comme les gels et les cires.

Toutefois, ces formulations capillaires de coiffage et surtout les sprays et les laques aérosols ne permettent encore pas à la coiffure de résister de manière satisfaisante aux différents mouvements naturels de la vie comme la marche, les mouvements de tête ou les coups de vent. En outre, ces compositions confèrent à la chevelure une sensation de raideur, appelée "effet casque". Comme produit coiffant, on connaît aussi les shampoings coiffants.

Les polymères classiquement utilisés pour la formulation des produits capillaires de coiffage sont des polymères filmogènes anioniques, amphotères ou non ioniques, qui conduisent à la formation de films possédant un caractère plus ou moins dur et cassant.

Lorsque le polymère est trop cassant, le pourcentage d'allongement à la rupture mesuré sur le film est faible, c'est-à-dire en général inférieur à 2 % et la tenue de la coiffure n'est pas assurée dans le temps.

Pour remédier à ce problème, on a déjà mélangé ces polymères avec des plastifiants et obtenu des revêtements plus souples et non friables. Toutefois, ces films sont déformables et plastiques, c'est-à-dire qu'après déformation, ils ne récupèrent que très peu de leur forme initiale. Si la tenue de la coiffure est améliorée, elle n'est pas encore satisfaisante puisque la forme de la coiffure évolue dans le temps.

On recherche donc des compositions cosmétiques pour le maintien et/ou la fixation de la coiffure qui procurent à la chevelure, outre une fixation durable, de bonnes propriétés cosmétiques, notamment de conditionnement du cheveu conférant un bon démêlage, de la douceur et un aspect agréable, non collant, ainsi qu'une facilité d'emploi.

Par ailleurs, on recherche aussi pour la peau, notamment du visage humain, et les phanères en particulier des cils et des sourcils, des produits ayant des propriétés de soin, de protection et/ou de conditionnement.

En outre, que se soit pour la chevelure ou la peau, on recherche encore des compositions cosmétiques aptes à former sur ces matières kératiniques un film souple qui suit les mouvements de la peau ou de la chevelure, sans effet de tiraillement, de lourdeur, ou de sensation rigide.

**EXPOSE DE L'INVENTION**

De manière surprenante et inattendue, le demandeur a découvert qu'il était possible de remédier aux problèmes techniques évoqués ci-dessus, en utilisant des polymères particuliers, dans une composition non rincée.

L'invention a pour objet une composition cosmétique non rincée comprenant, dans un milieu cosmétiquement acceptable, au moins un polymère filmogène élastomérique choisi de telle sorte que le film obtenu par séchage de ce polymère, à température ambiante et à un taux d'humidité relative de 55 %, présente un profil mécanique défini par au moins :

(a) un taux d'allongement à la rupture ($\varepsilon_r$) supérieur ou égal à 800 %,

(b) une recouvrance instantanée ($R_i$) au moins égale à 75 %, après un allongement de 150 %,

(c) une recouvrance ($R_{300}$) à 300 secondes supérieure à 80 %.

Cette composition permet notamment l'obtention d'un film souple, non cassant sur les matières kératiniques humaines, qui suit leurs mouvements.

Par "au moins un" polymère filmogène élastomérique, on entend au sens de l'invention, un ou plusieurs (2, 3 ou plus) polymères filmogènes élastomériques.

La composition selon l'invention procure notamment un conditionnement et/ou un soin des matières kératiniques humaines, en particulier des cheveux ainsi qu'un effet coiffant d'un très bon niveau (haute fixation) de la chevelure et une excellent tenue de la coiffure dans le temps ; elle est de préférence une composition capillaire non rincée dont, par exemple, un après-shampoing conditionneur (ou composition conditionnante) à appliquer après un shampoing, une coloration, une décoloration, un défrisage ou une permanente de la chevelure.

On entend par "composition non rincée" toute composition qui est formulée pour subsister après application sur les matières kératiniques humaines tels que les cheveux, la peau ou les cils.

La composition non rincée peut être sous toutes les formes classiques de compositions cosmétiques non rincées, par exemple sous forme de gels, lotion, crème, cire, mousse, tels que les après- shampoings, conditionneurs, lotions de rinçage des cheveux, à appliquer après ou avant l'application d'une composition de permanente, d'ondulation, de défrisage, de teinture des cheveux, et des associations de ceux-ci, des produits d'hygiène corporelle tels que des produits de soin du corps, du visage, du cou ou des yeux, par exemple sous forme de sérum, lotion, crème, gel ou de lait.

En particulier, la composition de l'invention est une mousse, un gel, une lotion, une crème capillaire qui présente un effet coiffant et peut également présenter un effet conditionneur. Par effet conditionneur des matières kératiniques, on entend un apport de démêlage, de lissage, de brillance ou de volume.

Avantageusement, la composition de l'invention est sous forme d'une mousse ou d'un gel de coiffage à appliquer sur cheveux secs ou humides.

Aussi, l'invention a encore pour objet une mousse ou un gel de coiffage des cheveux à utiliser sans rinçage, contenant un milieu cosmétiquement acceptable et au moins un polymère filmogène élastomérique tel que défini précédemment. Cette mousse ou gel peut être prise au doigt ou pulvériser sur les cheveux à l'aide d'un flacon-pompe.

Un autre objet de la présente invention se rapporte à un procédé de mise en forme ou de maintien de la coiffure comprenant la mise en oeuvre de cette composition.

Un autre objet de la présente invention se rapporte à un procédé cosmétique de soin et/ou de protection de la peau, y compris du cuir chevelu, et/ou des fibres kératiniques humaines comme les cheveux, les cils et les sourcils, comprenant l'application d'une composition cosmétique telle que définie précédemment, sur la peau et/ou lesdites fibres kératiniques humaines. Le soin de la peau peut notamment consister en la réparation de celle-ci contre les méfaits du temps, comme la sécheresse, les rides et ridules, la mollesse. La protection de la peau vise en particulier la protection de cette dernière contre les stimuli extérieurs comme le vent, la pollution, le rayonnement U. V.

Un autre objet de la présente invention se rapporte à un procédé cosmétique de conditionnement des matières kératiniques humaines et notamment de la peau, y compris du cuir chevelu, et/ou des fibres kératiniques humaines, comprenant l'application d'une composition cosmétique telle que définie précédemment, sur ces matières kératiniques.

Encore un autre objet de la présente invention se rapporte à l'utilisation de cette composition pour la fabrication de compositions cosmétiques destinées au soin et/ou à la protection des matières kératiniques humaines telles que la peau, les cheveux, les cils, les ongles. Plus spécialement, l'invention a encore pour objet l'utilisation de cette composition pour la fabrication d'un produit cosmétique capillaire, en vue de maintenir et/ou de fixer la coiffure.

## DESCRIPTION DETAILLEE DES MODES DE REALISATION DE L'INVENTION

Au sens de la présente invention, on entend par film obtenu par séchage à température ambiante (22°C $\pm$ 2°C) et à un taux d'humidité relative de 55 % $\pm$ 5%, le film obtenu dans ces conditions à partir d'un mélange à 6 % de matière active (m.a.) de polymère filmogène élastomérique dans un mélange 30 % en poids d'éthanol et de 70 % en poids d'eau, par rapport au poids total alcool+eau, la quantité de mélange étant adaptée pour obtenir dans une matrice en téflon, un film d'épaisseur de 500 $\mu$m $\pm$ 50 $\mu$m. Le séchage est poursuivi jusqu'à ce que le poids du film n'évolue plus, ce qui représente environ 12 jours. Les polymères filmogène solubles ou partiellement solubles dans l'éthanol sont testés dans l'éthanol seul. Les autres polymères sont testés dans l'eau seule, sous forme soluble ou dispersée.

Au sens de la présente invention, le taux d'élongation à la rupture et le taux de recouvrance sont évalués aux moyens des essais décrits ci-après.

Pour effectuer les essais de traction, le film est découpé en éprouvettes de forme rectangulaire, de longueur 80 mm et de largeur 15 mm.

Les essais sont réalisés sur un appareil commercialisé sous l'appellation Lloyd ou commercialisé sous l'appellation Zwick dans les mêmes conditions de températures et d'humidité que pour le séchage, c'est-à-dire une température de 22°C $\pm$ 2 °C et un taux d'humidité relative de 50 % $\pm$ 5 %.

Les éprouvettes sont étirées à la vitesse de 20 mm/min et la distance entre les mors est de 50 $\pm$ 1 mm.

Pour déterminer la recouvrance instantanée ($R_i$), on procède comme suit :

- on étire l'éprouvette de 150 % ($\varepsilon_{max}$) c'est-à-dire 1,5 fois sa longueur initiale ($l_0$),
- on relâche la contrainte en imposant une vitesse de retour égale à la vitesse de traction, soit 20 mm/min, et on mesure l'allongement de l'éprouvette en pourcentage, après retour à charge nulle ($\varepsilon_i$).

La recouvrance instantanée en % ($R_i$) est donnée par la formule ci-après :

$$R_i = ((\varepsilon_{max} - \varepsilon_i)/ \varepsilon_{max}) \times 100$$

Pour déterminer la recouvrance à 300 secondes, on maintient à contrainte nulle pendant 300 secondes supplémentaires, l'éprouvette ayant subi les opérations précédentes, et on mesure son taux d'allongement en pourcentage ($\varepsilon_{300s}$).

La recouvrance à 300 secondes en % ($R_{300s}$) est donnée par la formule ci-après :

$$R_{300s} = ((\varepsilon_{max} - \varepsilon_{300s})/ \varepsilon_{max}) \times 100$$

De façon avantageusement, le ou les polymères de la composition selon l'invention, éventuellement associé (s) à un agent plastifiant et/ou un agent de filmification, sont tels qu'ils forment, dans les conditions des tests ci-dessus, un film d'allongement à la rupture allant de 800 % à 3000 % ; de recouvrance instantanée de 75 % à 100 % ; et une recouvrance à 300secondes allant de 85 % à 100 %.

Dans les compositions conformes à l'invention, le polymère filmogène élastomérique ou le mélange de polymères filmogènes élastomériques est, de préférence, présent à une concentration allant de 0,05 % à 20 % en poids, plus préférentiellement de 0,1 % à 15 % en poids, et par exemple de 0,25 % à 10 % en poids par rapport au poids total de la composition.

De manière avantageuse, le polymère filmogène élastomérique est choisi dans le groupe comprenant les polyuréthanes, les alcools polyvinyliques, les polymères comprenant au moins un motif (méth)acrylique, leurs associations. Il peut se présenter sous forme d'homopolymère ou de copolymère. En particulier, il se présente sous forme non réticulée dans la composition.

Si nécessaire, la composition peut, en outre comprendre un agent plastifiant et/ou un agent facilitant la filmification du ou des polymères élastomériques sur les matières kératiniques, dont la fonction est de modifier les propriétés du ou des polymères élastomériques. Un tel agent auxiliaire de filmification peut être choisi parmi tous les composés connus de l'homme du métier comme étant susceptibles de remplir la fonction recherchée, et être notamment choisi parmi les agents plastifiants et les agents de coalescence. Le ou les polymères filmogènes élastomériques, éventuellement associés à un agent plastifiant et/ou un agent facilitant la filmification sont aptes à former un film, après évaporation du milieu cosmétique. Cette évaporation peut être faite à l'air libre ou en apportant de la chaleur, par exemple à l'aide d'un séchoir.

Comme exemple d'agent plastifiant et/ou facilitant la filmification sur les matières kératiniques, on peut utiliser ceux décrits dans le document FR-A-2 782 917. En particulier, cet agent est choisi parmi les plastifiants ou agents de coalescence usuels, tels que:

- les glycols et leurs dérivés tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther, le pentylène glycol,
- les esters de glycérol,
- les dérivés de propylène glycol et en particulier le propylène glycol phényléther, le propylène glycol diacétate, le dipropylène glycol butyléther, le tripropylène glycol butyléther, le propylène glycol méthyléther, le dipropylène glycol éthyléther, le tripropylène glycol méthyléther et le diéthylène glycol méthyléther, le propylène glycol butyléther,
- les esters d'acides notamment carboxyliques, tels que des citrates, des phtalates, des adipates, des carbonates, des tartrates, des phosphates, des sébaçates,
- leurs mélanges.

La quantité d'agent plastifiant et/ou d'agent de filmification peut être choisie par l'homme du métier sur base de ses connaissances générales, de manière à obtenir un système polymérique (polymères élastomériques + agent plastifiant et/ou agent de filmification) conduisant à un film ayant les propriétés mécaniques souhaitées, tout en conservant à la composition les propriétés cosmétiques recherchées. En pratique, cette quantité varie de 0,01 % à 25 % du poids total de la composition et mieux de 0,01 % à 15 %.

La composition selon l'invention contient, en outre, un milieu cosmétiquement acceptable. Par "milieu cosmétiquement acceptable", on entend un milieu non toxique et susceptible d'être appliqué sur le cuir chevelu et les cheveux d'êtres humains, qui peut être vendue sans prescription médicale.

Avantageusement, le milieu cosmétiquement acceptable de la composition comprend une phase hydrophile contenant de l'eau et/ou un ou plusieurs solvants cosmétiquement acceptables miscibles à l'eau tels que les monoalcools en $C_1$-$C_4$ et les polyols comme le glycérol, le diglycérol, le propylèneglycol, le diéthylène glycol, le sorbitol ou les éthers de glycol.

De préférence, le milieu cosmétiquement acceptable de la composition est constitué par de l'eau ou un ou plusieurs solvants cosmétiquement acceptables tels que des alcools ou des mélanges eau-solvant(s), ce ou ces solvants étant de préférence choisi parmi les alcools en $C_1$-$C_4$.

Parmi ces alcools, on peut citer l'éthanol, l'isopropanol, le t-butanol. L'éthanol est particulièrement préféré.

De préférence, le (ou les) polymères filmogènes élastomériques de l'invention sont solubles ou hydrodispersibles en milieu aqueux ou hydroalcoolique. En particulier, le ou les polymères filmogènes élastomériques sont solubles à au moins 10 g en matière active dans 90 g de milieu aqueux ou hydroalcoolique (contenant 70 % eau et 30 % éthanol), à température ambiante et pression atmosphérique.

De façon avantageuse, le film formé présente une faible sensibilité à l'eau, par exemple en atmosphère d'humidité relative de 30 % à 80%, c'est-à-dire que le film garde ses propriétés élastomériques pendant plusieurs heures. Il est souple, non cassant et suit bien les mouvements de la peau et/ou de la chevelure. En particulier, entre 30 % et 80 % d'humidité relative, le taux d'allongement à la rupture du film obtenu ne varie pas plus de 50 % ($\pm$ 400 %) et/ou sa recouvrance instantanée ne varie pas plus de 25 % (18,75 %) ; autrement dit, entre 30 % et 80 % d'humidité relative, le taux d'allongement à la rupture du film obtenu est compris entre 400 % et 1200 % et/ou sa recouvrance instantanée est comprise entre 57 % et 93%.

Le milieu cosmétiquement acceptable de la composition peut, en outre, contenir une phase huileuse contenant un ou plusieurs corps gras liquides à température ambiante (22°C) et pression atmosphérique, non miscibles à l'eau, appelées "huiles". Ces huiles peuvent être notamment des huiles émollientes notamment d'origine synthétique telles que les esters d'acides gras et/ou d'alcools gras, des huiles d'origine minérale ou végétale, ou encore des huiles de silicone à structure linéaire ou cyclique, modifiée ou non, ces huiles pouvant être volatiles ou non.

Il peut aussi comprendre au moins un tensioactif, ce tensioactif étant choisi parmi les tensioactifs non-ioniques, les tensioactifs anioniques, les tensioactifs cationiques, les tensioactifs amphotères, et leurs mélanges. En particulier, ce tensioactif est choisi parmi les tensioactifs non-ioniques, amphotères et leurs mélanges.

Comme agent tensioactif anionique utilisable dans la présente invention, on peut notamment mentionner les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylaryl-polyéthersulfates, les monoglycéride sulfates ; les alkylsulfonates, les alkylamide-sulfonates, les alkylarylsulfonates, les $\alpha$-oléfine-sulfonates, les paraffine-sulfonates, les alkylsulfosuccinates, les alkyléthersulfosuccinates, les alkylamidesulfosuccinates, les alkylsulfo-acétates, les acylsarcosinates et les acylglutamates, les groupes alkyle et acyle de tous ces composés comportant de 6 à 24 atomes de carbone et le groupe aryle désignant de préférence un groupe phényle ou benzyle, leurs mélanges.

On peut également utiliser comme agent tensioactif anionique, les monoesters d'alkyle en $C_6$-$C_{24}$ et d'acides polyglycoside-dicarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comportant de 12 à 20 atomes de carbone, leurs mélanges.

Un autre groupe d'agents tensioactifs anioniques utilisable dans la composition de la présente invention est celui des acyl-lactylates dont le groupe acyle comporte de 8 à 20 atomes de carbone.

En outre, on peut encore citer les acides alkyl-D-galactoside-uroniques et leurs sels ainsi que les acides alkyl ($C_{6-24}$)éther-carboxyliques polyoxyalkylénés, les acides alkyl($C_{6-24}$)aryl($C_{6-24}$)éther-carboxyliques poly-oxyalkylénés, les acides alkyl($C_{6-24}$) amidoéther-carboxyliques poly-oxyalkylénés et leurs sels, en particulier ceux comportant de 2 à 50 motifs oxyde d'éthylène, et leurs mélanges.

On utilise de préférence comme agent tensioactif anionique, les alkylsulfates, les alkyléthersulfates et les alkyléthercarboxylates, leurs mélanges, en particulier sous forme de sels de métaux alcalins ou alcalino-terreux, d'ammonium, d'amine ou d'aminoalcool.

Comme agent tensioactif amphotère utilisable dans la présente invention, on peut citer les dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer en particulier les alkyl($C_{8-20}$)bétaïnes, les sulfobétaïnes, les alkyl($C_{8-20}$)amidoalkyl($C_{6-8}$)-bétaïnes, les alkyl($C_{8-20}$)amidoalkyl($C_{6-8}$)sulfo-bétaïnes et leurs mélanges.

Parmi les dérivés d'amines, on peut citer les produits commercialisés sous la dénomination MIRANOL® , tels que décrits dans les brevets US 2 528 378 et US 2 781 354 et classés dans le dictionnaire CTFA, 3ème édition, 1982, sous les dénominations Amphocarboxy-glycinate et Amphocarboxypropionate de structures respectives (1) et (2) :

$$R_a\text{-CONHCH}_2\text{CH}_2\text{-N}(R_b)(R_c)(\text{CH}_2\text{COO}^-) \qquad (1)$$

dans laquelle :

. $R_a$ représente un groupe alkyle dérivé d'un acide $R_a$-COOH présent dans l'huile de coprah hydrolysée, un groupe heptyle, nonyle ou undécyle,
. $R_b$ représente un groupe bêta-hydroxyéthyle, et
. $R_c$ représente un groupe carboxyméthyle ; et

$$R_a'\text{-CONHCH}_2\text{CH}_2\text{-N}(B)(C) \qquad (2)$$

dans laquelle :

. B représente -$CH_2CH_2OX'$,
. C représente -$(CH_2)_z$-Y', avec z = 1 ou 2,
. X' représente le groupe -$CH_2CH_2$-COOH ou un atome d'hydrogène,
. Y' représente -COOH ou le groupe -$CH_2$-CHOH-$SO_3H$,
. $R_a'$ représente un groupe alkyle d'un acide $R_a'$-COOH présent dans l'huile de coprah ou dans l'huile de lin hydrolysée, un groupe alkyle, notamment en $C_{17}$ et sa forme iso, un groupe en $C_{17}$ insaturé.

Ces composés sont classés dans le dictionnaire CTFA, 5éme édition, 1993, sous les dénominations coco-amphodiacétate de disodium, lauro-amphodiacétate de disodium, caprylamphodiacétate de disodium, caprylo-amphodiacétate de disodium, coco-ampho-dipropionate de disodium, lauro-ampho-dipropionate de disodium, caprylampho-dipropionate de disodium, caprylo-ampho-dipropionate de disodium, acide lauro-ampho-dipropionique, acide coco-ampho-dipropionique.

A titre d'exemple, on peut citer le cocoamphodiacétate commercialisé par la société RHODIA sous la dénomination commerciale MIRANOL® C2M concentré.

Parmi les agents tensioactifs amphotères, on utilise de préférence les (alkyle en $C_{8-20}$)-bétaïnes, les (alkyle en $C_{8-20}$)-amido(alkyle en $C_{6-8}$)bétaïnes, les alkylamphodiacétates et leurs mélanges.

Comme agent tensioactif non-ionique utilisable dans la composition selon l'invention, on peut citer les composés connus décrits notamment dans le livre "Handbook of Surfactants" par M.R. PORTER, éditions Blackie & Son (Glasgow and London), 1991, pp 116-178). Il est choisi notamment parmi les alcools, les alpha-diols, les alkyl($C_{1-20}$) phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30, leurs mélanges.

On peut également citer comme agent tensioactif non-ionique utilisable dans l'invention les condensats d'oxyde d'éthylène et d'oxyde de propylène sur des alcools gras ; les amides gras polyéthoxylés ayant de préférence de 2 à 30 motifs d'oxyde d'éthylène ; les amides gras polyglycérolés comportant en moyenne de 1 à 5 groupements glycérol et en particulier de 1,5 à 4 ; les esters d'acides gras du sorbitane éthoxylés ayant de 2 à 30 motifs d'oxyde d'éthylène ; les esters d'acides gras du saccharose ; les esters d'acides gras de polyéthylèneglycol ; les (alkyle en $C_{6-24}$) polyglycosides ; les dérivés de N-(alkyle en $C_{6-24}$)glucamine ; les oxydes d'amines tels que les oxydes d'(alkyle en $C_{10-14}$)amines ou les oxydes de N-(acyle en $C_{10-14}$)-aminopropylmorpholine ; leurs mélanges.

Parmi les tensioactifs non-ioniques cités ci-dessus, on utilise de préférence les (alkyle en $C_{6-24}$)polyglycosides.

La quantité totale d'agent tensioactif est généralement comprise dans la gamme allant de 0,01 % à 50% en poids, de préférence de 0,1 % à 25 % en poids par rapport au poids total de la composition.

La composition de l'invention peut également contenir au moins un ingrédient additionnel classiquement utilisé dans le domaine cosmétique et choisi parmi les principes les additifs de formulation et les actifs cosmétiques à effet bénéfique sur les matières kératiniques comme la peau et les fibres kératiniques telles les cheveux.

Comme additif de formulation, on peut citer les épaississants de phase aqueuse ou de phase grasse, les polymères fixants ou non, anioniques, non ioniques, cationiques ou amphotères, les cires, les gommes, les charges, les parfums, les conservateurs, les agents séquestrant, les agents de modification de pH tels que des solutions basiques (par exemple des solutions d'hydroxyde de sodium, de potassium, d'ammonium, les aminométhylpropanols et les amines primaires, secondaires et tertiaires) ou des solutions tampons (par exemple du bicarbonate de sodium), ou encore les solutions acides (par exemple des solutions d'acide citrique ou chlorhydrique), les agents nacrants, les matières colorantes et les précurseurs de coloration, les agents opacifiants et tout autre additif classiquement utilisé dans les compositions cosmétiques destinées à être appliquées sur les matières kératiniques telles que la peau, les cheveux, les cils.

Comme épaississant de phase aqueuse, on peut citer des polymères comme les polymères ou copolymères carboxyliques (Carbopol®), les dérivés de cellulose, tels que la carboxyméthylcellulose, l'ydroxypropylcellulose, l'hydroxyéthylcellulose, la gomme de guar et les gommes de guar hydroxypropylées ; des électrolytes comme le chlorure de sodium ; des hydrotopes comme le toluène sulfonate de sodium ou de potassium ou le xylènesulfonate de sodium ; leurs mélanges.

La composition de l'invention peut également contenir au moins un actif cométique à effet bénéfique tels que les sels de zinc d'acide organique (acétate, glycolate, lactate, gluconate ou citrate) ou minéral (chlorure et le sulfate), les filtres solaires, les protéines, les vitamines (A, E, B$_2$, B$_5$, F, C), les provitamines, les céramides, les pseudocéramides, les agents anti-radicalaires, les extraits de végétaux.

Les quantités des différents ingrédients additionnels de la composition selon l'invention sont celles généralement utilisées dans les domaines considérés et sont en particulier comprises dans la gamme allant de 0,001 % à 20% du poids total de la composition. En outre, cette composition est préparée selon les méthodes usuelles.

Ces compositions peuvent être conditionnées sous diverses formes, notamment dans des flacons éventuellement pourvus de pompe, des bouillottes, des tubes ou de simples pots pour une prise à la main. Les compositions conformes à l'invention peuvent se présenter sous la forme de crème, de gel, d'émulsion eau-dans-huile ou huile-dans-eau, de lotion, de spray, de mousse ou de cire.

Dans un mode de mise en oeuvre, le pH de la phase aqueuse est choisi dans la gamme allant de 2 à 11 et préférentiellement de 3 à 10 par exemple de 5 à 8.

Avantageusement, la composition présente une viscosité dynamique, mesurée à température ambiante et pression atmosphérique inférieure à 200 cps, et par exemple comprise entre 180 cps et 10 cps. Cette viscosité peut être mesurée par toute méthode connue et par exemple à l'aide d'un Rhéomat 180, notamment à 25°C et sous un taux de cisaillement de 1 s$^{-1}$.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas ou substantiellement pas, altérées par l'addition envisagée.

Les compositions conformes à l'invention peuvent être appliquées sur des matières kératiniques tels que les cheveux, peau, et cils à l'état sec ou humide.

A titre illustratif et non limatif, on donne ci-après des exemples concrets de réalisation de la composition selon l'invention.

Les quantités sont données en pourcentage massique et M.A. signifie matière active.

**EXEMPLES**

**Exemple 1 : Mousse de coiffage**

On prépare la composition suivante par simple mélange à froid (température ambiante) des différents constituants, dans un flacon pressurisé.

| | |
|---|---|
| Polyuréthane (NMDEA[1] / PTMO 2900[2] /IPDI[3] - -3/1/4) | 2 % MA |
| Monolaurate de sorbitane oxyéthyléné | 0,2 % MA |
| Eau | 92,8 % MA |
| Isobutane / propane (85/15) | 5 % MA |
| [1] - N-méthyldiéthanolamine [2] - Poly (tétraméthylène oxyde) ayant une masse moyenne en poids de 2900 [3] - Isophoronediisocianate | |

Le copolymère de polyuréthane peut être préparé comme décrit dans le document FR-A-2 815 350 et en particulier selon le procédé de synthèse du polyuréthane PU2 de l'exemple 1, en remplaçant le poly(tétraméthylène oxyde) de masse moléculaire moyenne en poids de 1400 par un poly (tétraméthylène oxyde) de masse moléculaire moyenne en poids de 2900.

Le copolymère de polyuréthane présente les caractéristiques suivantes, mesurées comme ci-dessus :

- $\varepsilon_r$ = 1500 %
- $R_i$=82%
- $R_{300}$ = 92 %
- Soluble dans l'eau à au moins 10 g par litre

Cette composition peut être appliquée sur cheveux secs ou humides. Elle confère à la coiffure de bonnes propriétés coiffantes (bon niveau de fixation et très bonne tenue de la coiffure dans le temps), qui suit les mouvements de la chevelure.

**Exemple2 : Gel de coiffage**

On prépare la composition suivante par simple mélange à froid des différents constituants.

| Polyuréthane (NMDEA[1] / PTMO 2900[2] / IPDI[3]- 3 / 1 / 4) | 4 % MA |
|---|---|
| Jaguar HP 105 (Rhodia) | 1 % MA |
| Eau | qsp 100% MA |

Ce gel de coiffage procure aux cheveux de bonnes propriétés coiffantes (bon niveau de fixation et très bonne tenue de la coiffure dans le temps, souple), sans effet casque.

**Revendications**

1. Composition cosmétique non rincée comprenant, dans un milieu cosmétiquement acceptable, au moins un polymère filmogène élastomérique choisi de telle sorte que le film obtenu par séchage de ce polymère, à température ambiante et à un taux d'humidité relative de 55 %, présente un profil mécanique défini par au moins:

   (a) un taux d'allongement à la rupture ($\varepsilon_r$) supérieur ou égal à 800 %,
   (b) une recouvrance instantanée ($R_i$) au moins égale à 75 %, après un allongement de 150 %,
   (c) une recouvrance ($R_{300}$) à 300 secondes supérieure à 80 %.

2. Composition selon la revendication 1, **caractérisée en ce que** le polymère filmogène élastomérique est soluble dans un milieu aqueux ou hydroalcoolique.

3. Composition selon la revendication 1 ou 2, **caractérisée en ce qu'**entre 30 % et 80 % d'humidité relative, le taux d'allongement à la rupture du film obtenu est compris entre 400 % et 1200 % et/ou sa recouvrance instantanée est comprise entre 57 % et 93%.

4. Composition selon quelconque l'une des revendications précédentes, **caractérisée en ce qu'**elle contient au moins un tensioactif.

5. Composition selon la revendication précédente, **caractérisée en ce que** la quantité totale d'agent tensioactif est comprise dans la gamme allant de 0,01 % à 50% en poids, de préférence de 0,1 % à 25 % en poids par rapport au poids total de la composition.

6. Composition selon l'une des revendications précédentes, **caractérisée en ce que** la composition contient au moins un agent tensioactif choisi parmi les agents tensioactifs non-ioniques, amphotères et leurs mélanges.

7. Composition selon l'une des revendications précédentes, **caractérisée en ce que** la composition est sous forme d'une mousse ou d'un gel de coiffage des cheveux.

8. Composition selon l'une des revendications 6 à 7, **caractérisée en ce que** la composition présente une viscosité dynamique, mesurée à température ambiante et pression atmosphérique inférieure à 200 cps.

9. Composition selon l'une quelconque des revendications précédentes, **caractérisée en ce que** le polymère filmogène élastomérique ou le mélange de polymères filmogènes élastomériques est présent à une concentration allant de 0,05 % à 20 % en poids, plus préférentiellement de 0,1 % à 15 % en poids, et par exemple de 0,25 % à 10 % en poids par rapport au poids total de la composition.

**10.** Composition selon l'une quelconque des revendications précédentes, **caractérisée en ce que** le polymère filmogène élastomérique est choisi dans le groupe comprenant les polyuréthanes, les alcools polyvinyliques, les polymères comprenant au moins un motif (méth)acrylique, leurs associations.

**11.** Composition selon l'une quelconque des revendications précédentes, **caractérisée en ce que** le milieu cosmétiquement acceptable comprend une phase hydrophile contenant de l'eau et/ou un ou plusieurs solvants cosmétiquement acceptables miscibles à l'eau.

**12.** Composition selon la revendication précédente, **caractérisée en ce que** le solvant est choisi parmi les alcools en $C_1$-$C_4$.

**13.** Composition selon l'une des revendications précédentes, **caractérisée en ce que** la composition contient au moins un ingrédient additionnel choisi parmi les principes actifs cosmétiques et les additifs de formulation.

**14.** Procédé cosmétique de conditionnement des matières kératiniques humaines, comprenant l'application d'une composition cosmétique non rincée telle que définie à l'une des revendications précédentes, sur les matières kératiniques humaines.

**15.** Procédé cosmétique de soin et/ou de protection de la peau, y compris du cuir chevelu, et/ou des fibres kératiniques humaines, comprenant l'application d'une composition cosmétique telle que définie à l'une des revendications 1 à 13, sur la peau et/ou lesdites fibres kératiniques humaines.

**16.** Procédé de maintien ou de mise en forme de la coiffure, **caractérisé par le fait qu'**il comprend la mise en oeuvre d'une composition conforme à l'une quelconque des revendications 1 à 13.

**17.** Utilisation d'une composition selon l'une quelconque des revendications 1 à 13, pour la fabrication de compositions cosmétiques destinées au soin et/ou à la protection des matières kératiniques telles que la peau, les cheveux, les cils, les ongles.

**18.** Utilisation d'une composition selon l'une quelconque des revendications 1 à 13, pour la fabrication d'un produit cosmétique capillaire, en vue de maintenir et/ou de fixer la coiffure.

**Office européen des brevets**

**RAPPORT DE RECHERCHE EUROPEENNE**

Numéro de la demande

EP 05 29 1362

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | FR 2 848 430 A (OREAL) 18 juin 2004 (2004-06-18) * page 34, ligne 23-34; revendication 1 * ----- | 1-18 | A61K7/11 |
| X | FR 2 786 391 A (OREAL) 2 juin 2000 (2000-06-02) * revendications 1,10,11 * ----- | 1-18 | |
| X | FR 2 801 200 A (OREAL) 25 mai 2001 (2001-05-25) * revendications 1,7,8 * ----- | 1-18 | |
| X | FR 2 801 204 A (OREAL) 25 mai 2001 (2001-05-25) * page 11, ligne 11-18; revendications 1,12-14; tableau 1 * ----- | 1-18 | |
| X | FR 2 801 203 A (OREAL) 25 mai 2001 (2001-05-25) * page 11, ligne 11-18; revendications 1,12-14; tableau 1 * ----- | 1-18 | DOMAINES TECHNIQUES RECHERCHES (Int.Cl.7) |
| X | FR 2 815 350 A (OREAL) 19 avril 2002 (2002-04-19) * le document en entier * ----- | 1-18 | A61K |
| X | FR 2 821 621 A (OREAL) 6 septembre 2002 (2002-09-06) * revendications 1,4 * ----- | 1-18 | |
| X | FR 2 801 202 A (OREAL) 25 mai 2001 (2001-05-25) * revendications 1,7,8; exemples 1,2 * ----- | 1-18 | |
| X | FR 2 815 854 A (OREAL) 3 mai 2002 (2002-05-03) * page 8, ligne 24-31; revendications 1,7-9; exemples 1-3 * ----- | 1-18 | |

-/--

Le présent rapport a été établi pour toutes les revendications

| Lieu de la recherche | Date d'achèvement de la recherche | Examinateur |
|---|---|---|
| Munich | 21 octobre 2005 | Yon, J-M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

................................................................
& : membre de la même famille, document correspondant

EPO FORM 1503 03.82 (P04C02)

**Office européen**

**des brevets**

**RAPPORT DE RECHERCHE EUROPEENNE**

Numéro de la demande

EP 05 29 1362

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | FR 2 786 392 A (OREAL) 2 juin 2000 (2000-06-02) * page 12, ligne 9-15; revendications 1,9,10; tableau 1 * ----- | 1-18 | |

DOMAINES TECHNIQUES RECHERCHES (Int.Cl.7)

Le présent rapport a été établi pour toutes les revendications

| Lieu de la recherche | Date d'achèvement de la recherche | Examinateur |
|---|---|---|
| Munich | 21 octobre 2005 | Yon, J-M |

CATEGORIE DES DOCUMENTS CITES

X : particulièrement pertinent à lui seul
Y : particulièrement pertinent en combinaison avec un
   autre document de la même catégorie
A : arrière-plan technologique
O : divulgation non-écrite
P : document intercalaire

T : théorie ou principe à la base de l'invention
E : document de brevet antérieur, mais publié à la
   date de dépôt ou après cette date
D : cité dans la demande
L : cité pour d'autres raisons

& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 05 29 1362

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

21-10-2005

| Document brevet cité au rapport de recherche | | Date de publication | Membre(s) de la famille de brevet(s) | | Date de publication |
|---|---|---|---|---|---|
| FR 2848430 | A | 18-06-2004 | BR | 0305902 A | 17-05-2005 |
| | | | CN | 1506035 A | 23-06-2004 |
| | | | EP | 1444974 A2 | 11-08-2004 |
| | | | JP | 2004196801 A | 15-07-2004 |
| FR 2786391 | A | 02-06-2000 | AU | 745846 B2 | 11-04-2002 |
| | | | AU | 1276700 A | 13-06-2000 |
| | | | BR | 9907723 A | 17-10-2000 |
| | | | CN | 1295459 A | 16-05-2001 |
| | | | EP | 1051146 A1 | 15-11-2000 |
| | | | WO | 0030594 A1 | 02-06-2000 |
| | | | JP | 2002530310 T | 17-09-2002 |
| | | | JP | 2004307516 A | 04-11-2004 |
| | | | PL | 341980 A1 | 07-05-2001 |
| | | | RU | 2183449 C2 | 20-06-2002 |
| | | | US | 6391292 B1 | 21-05-2002 |
| FR 2801200 | A | 25-05-2001 | AU | 1710601 A | 30-05-2001 |
| | | | BR | 0015691 A | 09-07-2002 |
| | | | EP | 1233739 A1 | 28-08-2002 |
| | | | WO | 0135910 A1 | 25-05-2001 |
| | | | JP | 2003533433 T | 11-11-2003 |
| | | | MX | PA02004994 A | 18-09-2002 |
| FR 2801204 | A | 25-05-2001 | AU | 768024 B2 | 27-11-2003 |
| | | | AU | 1711201 A | 30-05-2001 |
| | | | BR | 0008909 A | 04-12-2001 |
| | | | CA | 2360430 A1 | 25-05-2001 |
| | | | CN | 1336815 A | 20-02-2002 |
| | | | CZ | 20012513 A3 | 13-02-2002 |
| | | | EP | 1146847 A1 | 24-10-2001 |
| | | | WO | 0135911 A1 | 25-05-2001 |
| | | | JP | 2003513999 T | 15-04-2003 |
| | | | MX | PA01007255 A | 09-04-2002 |
| | | | PL | 348862 A1 | 17-06-2002 |
| | | | RU | 2219900 C2 | 27-12-2003 |
| | | | US | 6749837 B1 | 15-06-2004 |
| FR 2801203 | A | 25-05-2001 | AU | 1711301 A | 30-05-2001 |
| | | | BR | 0015762 A | 06-08-2002 |
| | | | CA | 2391640 A1 | 25-05-2001 |
| | | | CN | 1391462 A | 15-01-2003 |
| | | | EP | 1233741 A1 | 28-08-2002 |
| | | | WO | 0135915 A1 | 25-05-2001 |
| | | | JP | 2003527339 T | 16-09-2003 |
| | | | MX | PA02004996 A | 18-09-2002 |

EPO FORM P0460

Pour tout renseignement concernant cette annexe : voir Journal Officiel de l'Office européen des brevets, No.12/82

**EP 1 632 221 A1**

**ANNEXE AU RAPPORT DE RECHERCHE EUROPEENNE
RELATIF A LA DEMANDE DE BREVET EUROPEEN NO.**

EP 05 29 1362

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

21-10-2005

| Document brevet cité au rapport de recherche | | Date de publication | Membre(s) de la famille de brevet(s) | | Date de publication |
|---|---|---|---|---|---|
| FR 2801203 | A | | PL | 356209 A1 | 14-06-2004 |
| FR 2815350 | A | 19-04-2002 | AU | 9569201 A | 29-04-2002 |
| | | | EP | 1326908 A1 | 16-07-2003 |
| | | | WO | 0232978 A1 | 25-04-2002 |
| | | | JP | 2004511637 T | 15-04-2004 |
| | | | US | 2004052753 A1 | 18-03-2004 |
| FR 2821621 | A | 06-09-2002 | EP | 1373346 A1 | 02-01-2004 |
| | | | WO | 02070577 A1 | 12-09-2002 |
| | | | JP | 2004529228 T | 24-09-2004 |
| | | | US | 2004197293 A1 | 07-10-2004 |
| FR 2801202 | A | 25-05-2001 | AU | 747931 B2 | 30-05-2002 |
| | | | AU | 7148000 A | 24-05-2001 |
| | | | BR | 0005647 A | 19-06-2001 |
| | | | CA | 2325900 A1 | 19-05-2001 |
| | | | CN | 1300587 A | 27-06-2001 |
| | | | EP | 1101486 A1 | 23-05-2001 |
| | | | JP | 3669916 B2 | 13-07-2005 |
| | | | JP | 2001163722 A | 19-06-2001 |
| | | | JP | 2005139202 A | 02-06-2005 |
| | | | PL | 343950 A1 | 21-05-2001 |
| | | | RU | 2200533 C2 | 20-03-2003 |
| | | | US | 6432386 B1 | 13-08-2002 |
| FR 2815854 | A | 03-05-2002 | AU | 758105 B2 | 13-03-2003 |
| | | | AU | 7828901 A | 02-05-2002 |
| | | | BR | 0105690 A | 25-06-2002 |
| | | | CA | 2360899 A1 | 30-04-2002 |
| | | | CN | 1350837 A | 29-05-2002 |
| | | | CZ | 20013637 A3 | 12-06-2002 |
| | | | EP | 1201224 A1 | 02-05-2002 |
| | | | HU | 0104562 A2 | 28-11-2002 |
| | | | JP | 2002145727 A | 22-05-2002 |
| | | | MX | PA01010998 A | 12-08-2004 |
| | | | PL | 350444 A1 | 06-05-2002 |
| | | | US | 2002086040 A1 | 04-07-2002 |
| | | | ZA | 200108318 A | 02-05-2002 |
| FR 2786392 | A | 02-06-2000 | AU | 746140 B2 | 18-04-2002 |
| | | | AU | 1276600 A | 13-06-2000 |
| | | | BR | 9907724 A | 17-10-2000 |
| | | | CN | 1295458 A | 16-05-2001 |
| | | | EP | 1051145 A1 | 15-11-2000 |
| | | | WO | 0030593 A1 | 02-06-2000 |

Pour tout renseignement concernant cette annexe : voir Journal Officiel de l'Office européen des brevets, No.12/82

13

**ANNEXE AU RAPPORT DE RECHERCHE EUROPEENNE
RELATIF A LA DEMANDE DE BREVET EUROPEEN NO.**

EP 05 29 1362

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

21-10-2005

| Document brevet cité<br>au rapport de recherche | Date de<br>publication | Membre(s) de la<br>famille de brevet(s) | Date de<br>publication |
|---|---|---|---|
| FR 2786392 A | | JP 2002530309 T<br>PL 341932 A1<br>RU 2200534 C2<br>US 6497864 B1 | 17-09-2002<br>07-05-2001<br>20-03-2003<br>24-12-2002 |

EPO FORM P0460

Pour tout renseignement concernant cette annexe : voir Journal Officiel de l'Office européen des brevets, No.12/82